# EUROPÄISCHE PATENTANMELDUNG

(11) **EP 2 011 881 A1**
(43) Veröffentlichungstag der Anmeldung: **07.01.2009**
(21) Anmeldenummer: 07075554.1
(22) Anmeldetag: 03.07.2007
(51) Int. Cl.: C12Q 1/68, G01N 33/50, C12N 15/10

(54) **Androgenabhängige Genfusion bestehend aus dem 1f-Aromatase Promotor und einem Reportergen**

(71) Anmelder: Bayer Schering Pharma Aktiengesellschaft, 13353 Berlin (DE)
(72) Erfinder: Oberndorf, Maik, 99425 Weimar (DE); Patchev, Vladimir, 07749 Jena (DE)

(57) **Zusammenfassung**

Die vorliegende Erfindung betrifft ein androgenabhängiges 1-f-Aromatase Reportergen sowie ein Verfahren zur Herstellung des 1-f-Aromatase-Reportergens und dessen Verwendung in einem Verfahren zur Identifizierung von Liganden des Androgenrezeptors.

SEQUENCE LISTING

<110>  Schering AG

<120>  1-f-Aromatase

<130>  52571

<160>  1

<170>  PatentIn version 3.1

<210>  1
<211>  670
<212>  DNA
<213>  Homo sapiens

<400>  1
ggctcctctc cccaagtcaa accttacctt acttaaccga ttgtatttcc tccctcagag      60

gatgccatat ctcagtacag gagagaaaat agaaggtaga gagactttta cccaagcacc     120

ccctgaaccc caggtgtaca caactgaacc tgatgcagtg acaatcacgt tcacacataa     180

aacatctggc taaaggctaa gatcacttcg gatttccgac atacattttc ctaagcagtg     240

catttttctt taattttcct tagaaaaaga ctgtaaagta gccccacaat tcccacatct     300

tcatactcca ccctgcattc aagttttcct gggacaggta tctatgtgtg tgcatgaatc     360

tatttttacg gcatatgtct aggaccccct acgaggagcc aaagtttcag agagcccagc     420

aactatgtaa ctccatggaa gggaggcatg atattactct ctgttcacag gagcgtacgc     480

acagatcttt tctcctcctc atggtcagtt ttctatttgt gattagtaat tagcttctct     540

tggtacgcta cgatctatta caaaagccaa acattcaggg ggcgagctga aatgacaaaa     600

tttggctata atttatgttg gcccctgaca tatatatttt tttaatggtt tggtctctaa     660

gcaactgatc                                                            670

## Beschreibung

Die vorliegende Erfindung betrifft ein androgenabhängiges 1-f-Aromatase Reportergen sowie ein Verfahren zur Herstellung des 1-f-Aromatase-Reportergens und dessen Verwendung in einem Verfahren zur Identifizierung von Liganden des Androgenrezeptors.

Die im zentralen Nervensystem durch Androgene induzierte Aromataseexpression und damit die Bildung von Estrogenen ist außerordentlich wichtig bei der Etablierung von geschlechtsspezifischen Verhaltensweisen, einschließlich der Prägung der Geschlechtsidentität, sowie der Steuerung der Libido, insbesondere im männlichem Geschlecht. Daneben scheint die durch Androgene induzierte Expression der Aromatase im Gehirn eine wichtige Rolle bei Vorgängen wie Lernen und Gedächnis zu haben (Wickelgren, I.; Science, Vol. 276: 675-678, 1997). Auch bei neuroregenerativen Prozessen sind positive Effekte durch Estrogene beschrieben (Abe-Dohmae, et al., J. Neurochem., Vol. 67: 2087-2095, 1996; (I.Azcoitia et al., 2001, J. Neurobiol. 47: 318-329). Eine Induktion oder Heraufregulierung des Estrogenrezeptors und der neuronalen Estrogenproduktion scheint Teil eines Reparaturmechanismus bei verschiedenen neuronalen Verletzungen zu sein (Dubal et al., 1999, J Neurosci 19:6385-6393; Garcia-Segura et al., 1999, Neuroscience 89: 567-578; Peterson et al., 2001, J Neuroendocrinol 13: 317-323; Carswell et al., 2005, J.Steroid Biochem. Mol.Biol. 96:89-91). Estrogene stellen daher eine bedeutende pharmakologische Option zur Behandlung oder Vermeidung von neurodegenerativen Erkrankungen dar. Allerdings ist ihre Verwendung in beiden Geschlechtern aufgrund möglicher Nebeneffekte in anderen Organen eingeschränkt.

Derzeit stehen keine *in vitro* Testsysteme zur Verfügung, mit deren Hilfe, die durch Androgene induzierte Aromataseexpression und so die gehirnspezifische Synthese von Estrogenen ermittelt werden kann und mit deren Hilfe Substanzen identifiziert und charakterisiert werden können, die die Aromataseaktivität beeinflussen.

Aus dem Stand der Technik sind *in vivo* Untersuchungen mit den Endpunkten Estrogenkonzentration im Gehirn, Aromataseexpression im Gehirn, Aromataseaktivität in Gehirnproben oder Verhaltenstestung in beiden Geschlechtern bekannt.

Wie schon erwähnt, induzieren Androgene die Aromataseexpression, dies wird vermittelt durch ihre Bindung an den Androgenrezeptor (AR). Der AR kann entweder aktiviert oder inaktiviert werden. Beispiele für eine Aktivierung im Falle von Androgenmangel sind z.B. bei Sarcopenia, Hypogonadismus, altersbedingtem Hypogonadismus sowie Formen der Störung der Libido und bei der erektilen Dysfunktion im Mann zu finden.

Die pharmakologische Inaktivierung des AR ist wichtig z.B. bei benignen und malignen Erkrankungen der Prostata oder bei Krankheiten, die mit einem Androgenüberschuss zusammenhängen, z. B. bestimmte Formen der Akne, des Haarausfalls oder des Polycystischen Ovars und Hirsutismus in der Frau.

In der Regel ist jedoch nicht eine vollständige Aktivierung oder Deaktivierung des Androgenrezeptors wünschenswert, sondern eine gewebeselektive Wirkung. So ist z.B. im Falle des männlichen Hypogonadismus eine AR vermittelte Aktivierung der Libido, des Gemützustandes und eine knochen- oder muskelanabole Wirkung erwünscht, jedoch keine Aktivierung, sondern eher eine neutrale oder eine abschwächende Wirkung auf die Prostata oder die Haut. Im Falle der gewünschten Deaktivierung des AR, z.B. bei malignen Prostatakarzinomen oder Haarausfall, ist eine Deaktivierung des AR bezüglich Libido, Knochenmetabolismus etc. nachteilig (S.S.Wolf und M.Obendorf, 2004, "Selective androgen receptor modulators (SARMs)"; in E. Nieschlag & H. M. Behre: 'Testosterone' 3rd. Edition; Cambridge University press, ISBN 0521833809; 623-640).

Derartige gewebeselektive Androgene oder Antiandrogene können bislang nur unzureichend in Zellkultur oder *in vitro* basierenden Assaysystemen gesucht und charakterisiert werden. Dies trifft auch zu für die Bestimmung der Aromataseexpression.

Daraus ergibt sich die Aufgabe ein Verfahren bereitzustellen, mit dessen Hilfe pharmakologisch aktive Substanzen identifiziert und charakterisiert werden können, wobei die Substanzen dadurch gekennzeichnet sind, dass sie
- eine selektive Erhöhung der Aromataseexpression im ZNS bewirken und /oder
- die Aktivität des AR gewebsselektiv beeinflussen.

Die Aufgabe wird gelöst durch ein Verfahren, in dem durch Stimulation der Aromataseexpression in einem Reportergenassay Substanzen identifiziert werden können, die die Estrogensynthese selektiv im Gehirn regulieren und den Androgenrezeptor gewebsselektiv beeinflussen. Es konnte gezeigt werden, dass der 1f-Aromatasepromotor androgenabhängig reguliert wird.

Die menschliche Aromatase ist in einem Gen kodiert, welches auf dem Chromosom 15 liegt (Chen, S et al., (1988): Molec. Biol., Vol. 7: 27-38).

Das humane P450ₐᵣₒₘ-Gen *(CYP19)* umfasst einen 30 kb kodierenden Bereich und einen rund 93 kb regulatorischen Bereich (Bulun, S.E. et al., Semin Reprod Med. Vol. 22: 5-9, 2004). Das Transkript besteht aus 10 Exonen, wobei das Exon 1 nicht kodierend ist. Die kodierenden Exone II bis X werden in allen Geweben gleich exprimiert. Splicingvarianten in den kodierenden Regionen sind bislang im Menschen noch nicht gefunden worden.

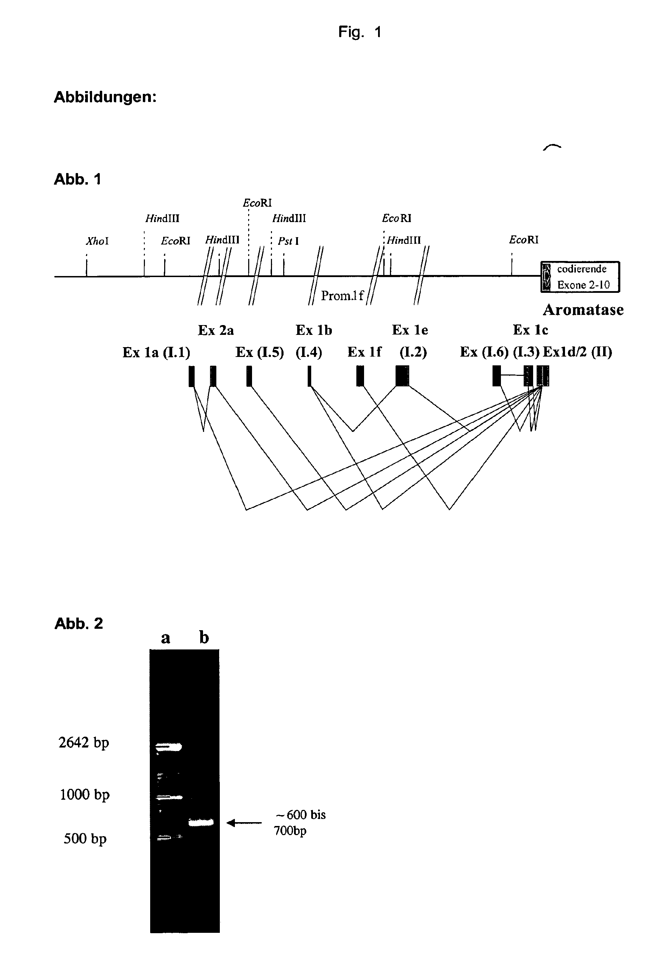

Für das Exon 1 wurden bei der Sequenzierung der mRNA aus verschiedenen Geweben mehrere Varianten entdeckt (zur Übersicht: Harada, N.; Utsumi, T. and Takagi, Y. Proc. Natl. Acad. Sci. USA, 90: 11312-11316, 1993, und Simpson, E. R.; Endocrine Reviews, 15: 341-355, 1994), die gewebespezifisch exprimiert und alle mit der selben am Beginn des Exons II liegenden Splicingstelle verknüpft werden (Abb. 1). Das gewebespezifische Auftreten der Exon-I-Sequenzen in der mRNA lässt sich durch die Verwendung alternativer Promotoren erklären, die im Gen vor den jeweiligen Exon-I-Varianten liegen. Die Aktivierung dieser verschiedenen Promotoren unterliegt einer noch nicht vollständig aufgeklärten Kontrolle.
Tabelle 1 zeigt eine beispielhafte Auflistung bereits bekannter Verwendungen bestimmter 5'-nichttranslatierter Exone der humanen Aromatase in den verschiedenen Geweben nach Harada et al. (1993, Proc. Natl. Acad. Sci. USA, Vol. 90: 11312-11316).

Bisherige Untersuchungen, insbesondere der beiden Arbeitsgruppen um Harada (Harada, N. et al., (1993) Proc. Natl. Acad. Sci. USA, Vol. 90: 11312-11316) und Simpson (Simpson, E. R.; Endocrine Reviews, Vol. 15: 341-355, 1994) haben durch Sequenzierung der Aromatase-mRNA ergeben, dass in den meisten Geweben mehrere Promotoren verwendet werden können und umgekehrt ein Promotor auch in unterschiedlichen Geweben genutzt werden kann (Tab. 1).

Eine Ausnahme scheint dabei der Promotor 1f darzustellen, der soweit bekannt, im Menschen ausschließlich in neuronalen Geweben benutzt wird.

Im Gehirn haben insbesondere Androgene einen Effekt auf die Expression der Aromatase (Abdelgadir, S. E. et al., Endocrinol., Vol. 135: 395-401, 1994; Rosselli, C. E. et al., (1996) Endocrine, Vol. 5: 59-65; Rosselli, C. E. et al., (1997, Brain Research, Vol. 44:351-357). Trotz dieser Erkenntnis ist bisher der genaue molekulare Mechanismus unbekannt, mit dem der Androgenrezeptor (AR) die Aromataseexpression im Gehirn reguliert. Es wird zwar ein putatives AR verantwortliches (responsible) Element im 1f-Aromatasepromotor in der Literatur erwähnt, aber eine funktionelle Involvierung des 1f-Promotors in der AR vermittelten Regulation der neuronalen Aromataseexpression wurde noch nicht gezeigt (Honda, S. et al., Biochem Biophys Res Commun 198 (1994), 1153-1160. Im Gegenteil: die Arbeitsgruppe, die den 1f-Aromatasepromotor zuerst beschrieben hat (S. Honda, et al., Biochem Biophys Res Commun. 198 (1994), 1153-1160), weist in späteren Veröffentlichungen auf andere nicht näher charakterisierte Regulatoren hin (Honda, S.l. et al., J Steroid Biochem Mol Biol. 79:255-60, 2001; Honda, S. et al., Brain Res Mol Brain Res. 66: 122-32, 1999).

Grundlage der vorliegenden Erfindung ist die Beobachtung, dass die Steuerung des 1f-Aromataseexons ausschließlich durch die Aktivitäten des 1f-Aromatasepromotors reguliert wird und nicht vom Zellkontext abhängig ist. Mittels eines *in vitro* Testsystems konnte nun der Einfluss von pharmakologisch aktiven Substanzen auf die Regulation der neuronalen Aromataseexpression, gezeigt werden, ohne dass neuronales Gewebe, neuronale Zellen, ganze Tiere oder Extrakte aus diesen verwendet werden müssen.

Mit Hilfe eines derartigen Testsystems wird das Auffinden und die Charakterisierung von pharmakologisch aktiven Substanzen ermöglicht und vereinfacht, die entweder die Erhöhung der Aromataseexpression selektiv im zentralen Nervensystem bewirken oder die geeignet sind, die Aktivität des AR gewebeselektiv zu beeinflussen.

Gegenstand der vorliegenden Erfindung ist daher ein *in vitro* Testsystem zur Identifizierung und Charakterisierung von Substanzen, die entweder die Erhöhung der Aromataseexpression selektiv im zentralen Nervensystem bewirken oder die geeignet sind, die Aktivität des AR gewebeselektiv zu beeinflussen, wobei das Testsystem eine Fusion aus Reportergen und Aromatasepromotor enthält.

Die gewebespezifische Regulation der Aromatase, d.h. des Schlüsselenzyms der Estrogensynthese, ist letztlich verantwortlich für die gezielte Bereitstellung von Estrogenen in den unterschiedlichen Geweben.

Zur Charakterisierung der hormongesteuerten Aromataseexpression sind etablierte humane Zell-Linien für solche *in vitro* Tests geeignet, wobei die Korrelation mit der gewebespezifischen *in vivo* Expression durch die Verwendung der unterschiedlichen Promotoren gewährleistet wird. Die Entwicklung eines Reportergenassays ist im Falle des Aromatasepromotors eine einfache und elegante Möglichkeit, den gewebespezifischen Einfluss bestimmter Substanzen auf die Expression durch Aktivierung eines einzelnen Promotors zu testen. Im Falle der gewünschten gehirnspezifischen Expression ist dies der erfindungsgemäße 1f-Aromatasepromotor.

Durch den Vergleich der Aktivität des 1f-Aromatasepromotors (1f-aro-promotor), der im neuronalen Gewebe durch den AR reguliert wird, mit der Aktivität eines Promotors, der in einem anderen Gewebe durch den AR reguliert wird oder generell durch AR reguliert wird, ist eine *in vitro* Charakterisierung von am AR aktiven Substanzen bezüglich Gewebeselektivität möglich. Dabei dient die Promotoraktivität des 1f-Aromatasepromotors als Surrogatmarker für eine AR vermittelte Aktivität in neuronalen Geweben oder noch generalisierter als Surrogatmarker für eine AR vermittelte Aktivität außerhalb der gonadalen Gewebe (z.B. im Mann außerhalb der Testis und Prostata, in der Frau außerhalb des Ovars).

Mittels eines Reportergenassays mit dem Promotor 1f der humanen Aromatase, kann die Regulation der neuronalen Aromataseexpression bestimmt werden.
Der neuronale 1f-Aromatase-Promotor, dessen Sequenz bekannt ist (Seq ID #1), wird vor ein Reportergen gesetzt. Die Promotor-Reportergenfusion wird transient oder stabil in eine Ziellinie hinein transfektiert. Die Expression der Luciferase erfolgt in Abhängigkeit des 1f-Promotors, die Aktivität des Aromatase-Promotors kann über die Menge des vom Reportergen kodierten Enzyms bestimmt werden. Als Reportergene wurden in den vorliegenden Beispielen das Firefly- und die Renilla- Luciferasegene exemplarisch ausgewählt.

Gegenstand der vorliegenden Erfindung ist eine Promotor-Reportergenfusion bestehend aus 1f-Aromatase-Promotor gemäß Seq ID #1 und Reportergen, wobei das Reportergen ausgewählt sein kann aus der Gruppe umfassend ein Firefly- oder Renilla-Luziferasegen, wobei diese Auswahl jedoch nicht einschränkend sein muss, und wobei das Fusionsprodukt stabil oder transient in eine Zelllinie exprimiert werden kann.

Wird zusätzlich der AR in den Zellen exprimiert, erfolgt der Einfluss der AR aktiven Testsubstanzen auf die Aktivität des Aromatasepromotors über den AR. Dabei kann die Wirkung der Testsubstanzen anhand der veränderten Luciferase-Enzymaktivitäten ermittelt werden. Damit können pharmakologisch aktive Substanzen gesucht oder charakterisiert werden, ohne dass mit primären neuronalen Gewebe oder primären neuronalen Zellen oder mit ganzen Tieren oder mit Extrakten daraus gearbeitet werden muss.

Gegenstand der vorliegenden Erfindung ist die Verwendung einer Promotor-Reportergenfusion in einem *in vitro* Testsystem, wobei die Promotor-Reportergenfusion aus einem 1f-Aromatase-Promotor gemäß Seq ID #1 und Reportergen besteht und wobei der Einfluss von Testsubstanzen in einem hochdurchsatzfähigen Reportergenassay in Zellkultur bestimmt wird und eine Aussage über die Regulation der Aromatase in neuronalen Geweben getroffen werden kann. Dieses Verfahren dient der Identifikation von Substanzen, die über Bindung an den Androgenrezeptor die Aromataseexpression beeinflussen.

Gegenstand der vorliegenden Erfindung ist auch die Verwendung der Promotor-Reportergenfusion, wobei Sequenzen, die >90%, vorzugsweise >95 % Homologie aufweisen zu der unter Seq ID # 1 angegebenen Sequenz, in einem Zellkultursystem zur Identifikation von Substanzen eingesetzt werden, die über Bindung an den Androgenrezeptor, die Aromataseregulation im Gehirn beeinflussen.

Des Weiteren können mit Hilfe des erfindungsgemäßen Testmodells auch die Eigenschaften androgener Substanzen, einschließlich AR Agonisten, Antagonisten und SARMs charakterisiert werden, in dem die Aktivität des 1f-Aromatasepromotors mit der Aktivität eines Promotors, der in einem anderen Gewebe durch den AR reguliert wird oder generell durch AR reguliert wird, verglichen wird. Dabei dient die Promotoraktivität des 1f-Aromatasepromotors als Surrogatmarker für eine AR vermittelte Aktivität in neuronalen Geweben oder noch generalisierter als Surrogatmarker für eine AR vermittelte Aktivität außerhalb der gonadalen Gewebe (z.B. im Mann außerhalb der Testis und Prostata, in der Frau außerhalb des Ovars).

Dazu wird das erfindungsgemäße Testsystem mit dem neuronalen 1f-Aromatase-promotor kombiniert mit einem anderen durch den AR regulierten Promotor. Letzterer spiegelt entweder eine allgemeine Androgenwirkung wider (z.B. der MMTV-Promotor) oder eine Androgenwirkung in einem anderen androgenen Zielorgan (z.B. Promotoren, die in der Prostata durch den Androgenrezeptor aktiviert werden). Die gleichzeitige Verwendung von zwei unterschiedlichen Reportergenen, also z.B die Renilla luciferase am 1f-Aromatasepromotor und die Firefly-Luciferase an einem zweiten Promotor, erlaubt die simultane Messung beider Promotoraktivitäten. Dies liefert einfach und elegant Auskünfte über die gewebeselektive Aktivität von zu testenden Substanzen, welche pharmakologisch über den AR wirken. Ansonsten können beide Promotoren auch sukzessiv vor dem selben Reportergen eingesetzt werden, was ebenfalls noch eine erhebliche Vereinfachung im Vergleich zum Stand der Technik, welches in der Regel ein Tierversuch ist, darstellt. Die neuronale Promotoraktivität kann generalisierter als Surrogataktivität eines Promotors außerhalb der Gonaden abstrahiert werden.

Ebenfalls Gegenstand der vorliegenden Erfindung ist ein Verfahren zur Bestimmung der gewebsselektiven Wirkung von Substanzen, die an den Androgenrezeptor binden, umfassend folgende Schritte:
a. Bestimmung der Modulation der Aromataseregulation unter Verwendung der Promotor-Reportergenfusion in einem Reportergenassay in Zellkultursystemen zur Identifikation von agonistischen wie auch antagonistischen Aktivitäten,
b. Bestimmung der Modulation eines anderen Androgen-abhängigen Promotors und
c. Vergleich von Schritt a und b, um eine vollständige *in vitro* Charakterisierung von Substanzen bezüglich selektiver AR Modulation am 1f-Aromatasepromotor oder im direkten Vergleich mit z.B. prostataspezifischen oder allgemein wirkenden AR regulierten Promotoren erlaubt. Dieses Verfahren dient der Identifikation von Substanzen, die den AR gewebsselektiv beeinflussen.
   Ebenfalls Gegenstand der vorliegenden Erfindung ist Verwendung der Promotor-Reportergenfusion in einem Reportergenassay, wobei Sequenzen, die > 90 %, vorzugsweise > 95 % Homologie aufweisen zu der unter Seq ID # 1 angegebenen Sequenz in einem Reportergenassay in Zellkultursystemen zur Identifikation von Substanzen eingesetzt werden, die die Aktivität des Androgenrezeptors modulieren und die Regulation von durch den Androgenrezeptor regulierten Genen gewebeselektiv modulieren.

Unter Antagonisten sind solche Moleküle zu verstehen, die an ihre entsprechenden Rezeptoren binden und welche die Initiierung des/der mit dem Rezeptor gekoppelten Signaltransduktionswege/s durch den oder die natürlich vorkommenden Liganden inhibieren. Üblicherweise kompetitieren die Antagonisten mit dem natürlich vorkommenden Liganden des Rezeptors um die Bindung an den Rezeptor. Aber auch andere Modifikationen des Rezeptors durch Moleküle, die verhindern, dass die mit dem Rezeptor gekoppelten Signaltransduktionswege durch den oder die natürlich vorkommenden Liganden aktiviert werden, sind möglich (z.B. nicht-kompetitive, sterische Modifikationen des Rezeptors).

Rezeptorantagonisten binden typischerweise selektiv an ihren bestimmten Rezeptor und nicht an andere Rezeptoren. Sie weisen normalerweise eine höhere Bindungsaffinität als der natürliche Ligand auf. Obwohl Antagonisten, die eine höhere Affinität zum Rezeptor haben als der natürliche Ligand, bevorzugt sind, können ebenfalls Antagonisten mit einer geringeren Affinität eingesetzt werden. Bevorzugterweise binden die Antagonisten reversibel an ihre korrespondierenden Rezeptoren.

Unter Agonisten sind solche Moleküle zu verstehen, die an ihre entsprechenden Rezeptoren binden und üblicherweise mit dem natürlich vorkommenden Liganden des Rezeptors um die Bindung an den Rezeptor kompetitieren und welche die Initiierung des mit dem Rezeptor gekoppelten Signaltransduktionsweges stimulieren. Agonisten können auch die Bindung des natürlichen Liganden unterstützen.

Rezeptoragonisten binden typischerweise selektiv an ihren bestimmten Rezeptor und nicht an andere Rezeptoren. Sie haben normalerweise eine höhere Bindungsaffinität als der natürliche Ligand. Obwohl Agonisten, die eine höhere Affinität zum Rezeptor haben als der natürliche Ligand, bevorzugt sind, können ebenfalls Agonisten mit einer geringeren Affinität eingesetzt werden. Bevorzugterweise binden die Agonisten reversibel an ihre korrespondierenden Rezeptoren.

Agonisten werden über die Initiierung der entsprechenden Rezeptor vermittelten Signaltransduktion und/oder physiologischen Wirkung getestet.

Als Liganden werden die Verbindungen oder niedermolekulare Substanzen bezeichnet, die an einen Rezeptor binden. Ihre Bindung ist üblicherweise reversibel. Durch die Bindung eines Liganden an den entsprechenden Rezeptor wird der mit dem Rezeptor gekoppelte Signaltransduktionsweg aktiviert oder inaktiviert. Auf diese Art und Weise vermittelt der Ligand seine intrazelluläre Wirkung.

Unter Liganden sind Agonisten und Antagonisten eines Rezeptors zu verstehen.

### Biologische Bespiele

### 1. Klonierung des humanen 1f-aromatase-promotors in Reportergenplasmide

Für den Reportergenassay wird der 1f-Promotor der humanen Aromatase aus genomischer DNA von humanen SH-SY5Y-Zellen amplifiziert. Die Isolierung von genomischer DNA erfolgt mit dem QlAamp Blood Kit nach dem vom Hersteller (Qiagen) vorgegebenen Protokoll. Der 1f-Promotor der Aromatase ist aus Veröffentlichungen (S. Honda, et al., Biochem Biophys Res Commun 198 (1994), pp. 1153-1160) und aus der Genbank (CYP19A1) bekannt. Unter Verwendung spezifischer Primerpaare (Tab. 2) werden Teile des humanen Aromatase-Promotorbereichs inklusive des 1f-Promotors mittels PCR amplifiziert.

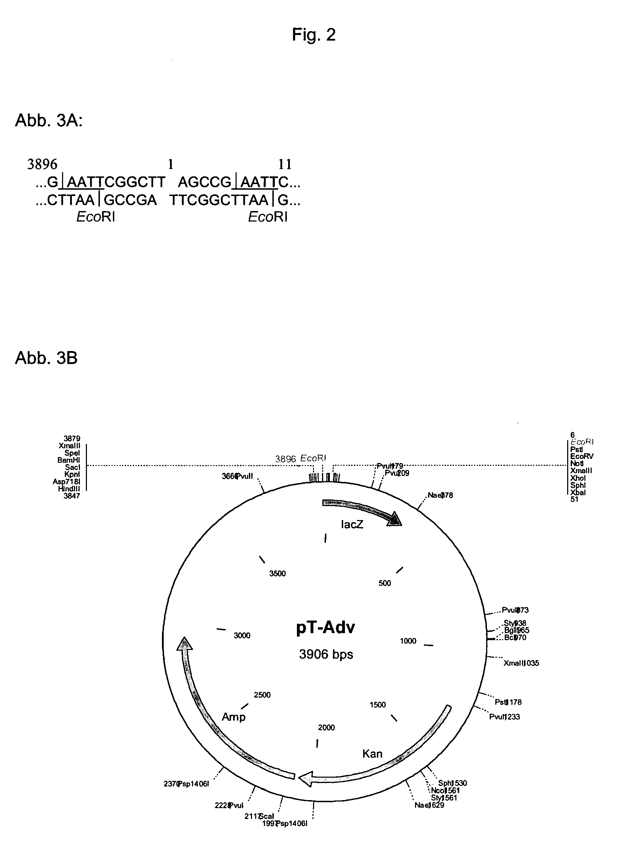

Zur Amplifikation von DNA-Fragmenten mittels PCR werden je 1 µl der genomischen DNA-Proben eingesetzt, für 2 min bei 94°C denaturiert und mit den angegebenen DNA-Primern EbrainB4 und Ebrain-c670 in 35 Zyklen vervielfältigt, gelelektrophoretisch analysiert (Abb. 2) und mittels DNA-Extraktion aus dem Agarosegel von Nebenprodukten gereinigt.

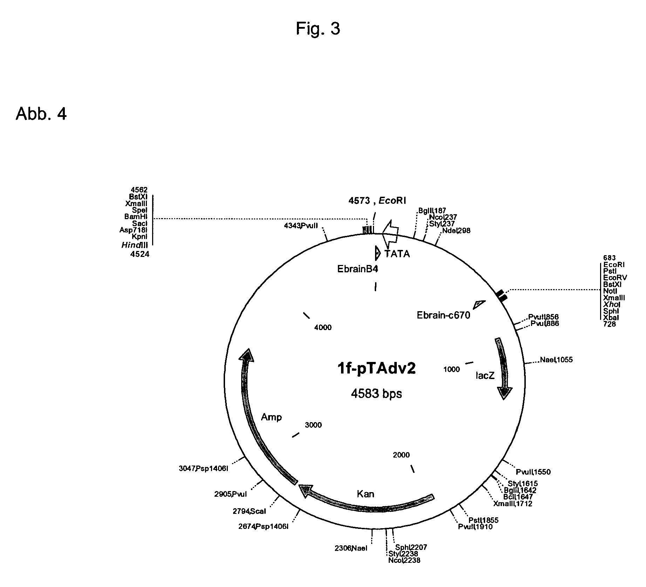

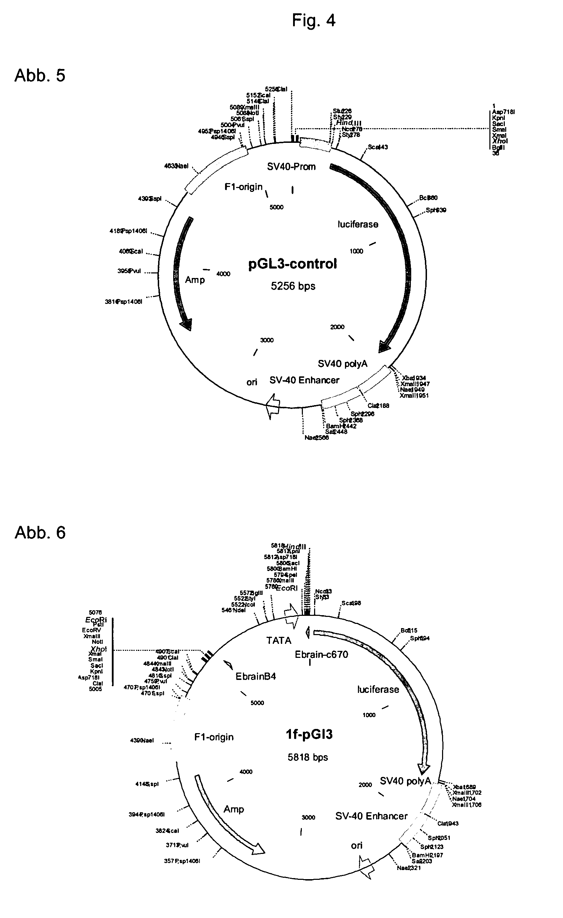

Das PCR-Produkt des 1f-Promotorfragmentes verfügt über 3'-A-Überhänge und wird mit dem linearen pTAdv-Klonierungsvektor (Abb. 3) ligiert. Der pTAdv-Vektor wurde von Clontech bezogen. Das Insert des entstandenen 1f-pTAdv2-Vektors (Abb. 4) wird sequenziert und die amplifizierte humane 1f-Aromatasepromotorsequenz bestätigt (Seq ID #1). Sie weicht von der zuvor veröffentlichten Sequenz ab (S. Honda et al., N Biochem Biophys Res Commun 198 (1994), 1153-1160), ist jedoch in Übereinstimmung mit neueren Genbankeinträgen.

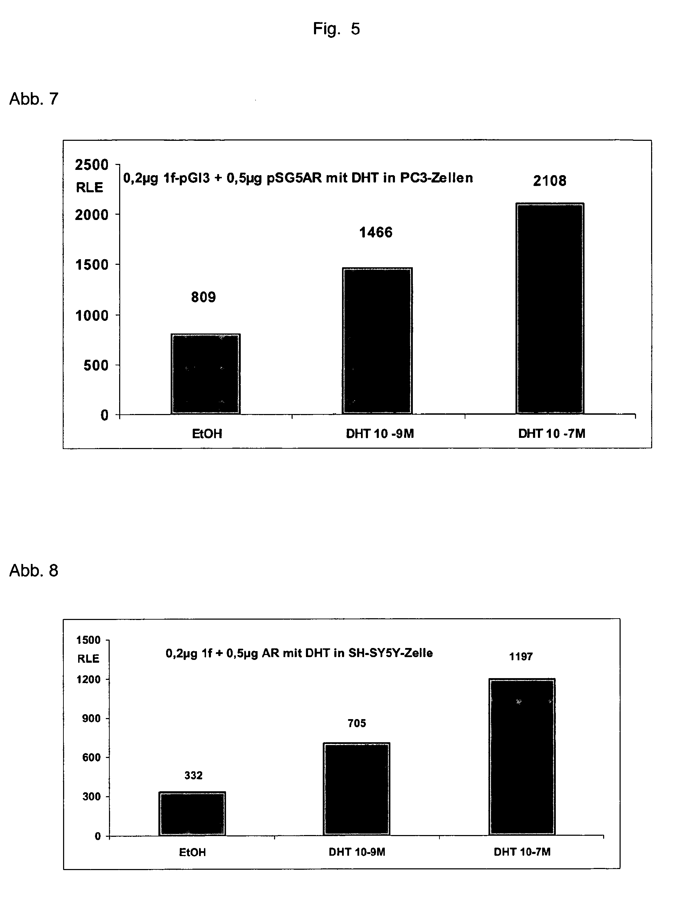

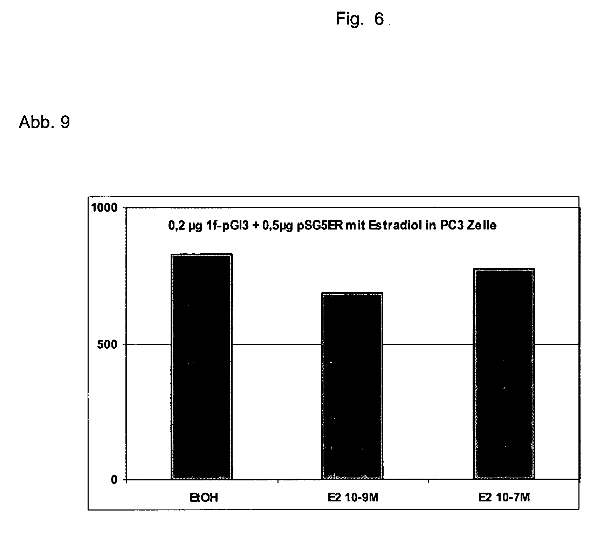

Als Reportergen wird z.B. das Luciferasegen des pGl3-Vektors von Promega verwendet. Für die Erzeugung des Promotor-Reportergen-Konstruktes mit dem 1f-Promotor, wird aus dem Vektor pGl3-control (Abb. 5) mit Hindlll und Xhol der Kontrollpromotor (SV40) mit 213 bp Länge herausgeschnitten. Der so linearisierte und verkürzte pGl3-Vektor wird durch Gelelektrophorese und DNA-Extraktion gereinigt, dephosphoryliert, anschließend wird das Fragment des 1f-Promotors, das mit HindIII und Xhol aus dem 1f-pTAdv2-Vektor (Abb. 4) herausgeschnitten und gereinigt wurde, hineinligiert und das Reportergenplasmid 1f-pGl3 erzeugt (Abb. 6).

### 2. Transfektionsexperimente zur Bestimmung des Einflusses hormonell aktiver Verbindungen auf den 1f-Promotor in Reportergenassays in Zellkultur

PC3 (humane prostata carcinoma)-Zellen (von DSMZ GmbH, Braunschweig) werden in RPMI 1640 Medium mit Phenolrot (Bst.Nr.: 31870-025 von Gibco) + 10 % fötales Kälberserum (FKS) mit 200 mM Glutamin und 5 mg/l Penicillin/Streptomycin passagiert und bei 37°C mit 5% CO₂ und 100% relativer Luftfeuchte in Brutschränken (Heraeus) kultiviert. Vor den Testungen werden die PC3-Zellen zweimal mit Testmedium passagiert, dieses enthält kein Phenolrot (Bst.Nr.: 32404-014 von Gibco) und fötales Kälberserum, welches zuvor über Aktivkohle von Hormonresten gereinigt wurde (DCS).

Um den Einfluss von Hormonen auf den klonierten 1f-Aromatasepromotor in dem pGl3-Fusions-vektor 1f-pGl3 anhand der Luciferase-Aktivität zu bestimmen, werden in 6well Platten pro well 200000 Zellen mit 0,2µg dieses Plasmides mit 0,5 µg des pSG5AR-Plasmid cotransfektiert. Der pSG5AR Vektor basiert auf dem pSG5-Vektor, der über den SV40-early-gene-Promotor verfügt, der in humanen Zellen zur konstitutiven Expression der dahinter liegenden Gene führt (Breathnach, R. und Harris, B.A. 1983, Nucleic Acids Res. 11:7119-36), in diesem Fall des Gens für den AR. Die transiente Transfektion mit dem Reportergenplasmid und dem pSG5AR-plasmid erfolgt mit Lipofectin nach den Angaben von GibcoBRL [Lipofectin-Reagent Packungsbeilage, Kat.Nr.: 18292-037]. 20 h nach der Transfektion wird das Androgen Dihydrotestosteron (DHT) zugegeben. Nach weiteren 24 Stunden Inkubation im Brutschrank werden die Zellen mit PBS gewaschen, lysiert und die Reportergenaktivität bestimmt. Die Bestimmung der Luciferaseaktivität erfolgt mit Luciferase-Assay-Substrat nach den Angaben von Promega [Luciferase-Assay-Substrat Packungsbeilage, Kat.Nr.: E151A aus: Luciferase Assay System with Reporter Lysis Buffer; Part TB161, USA 3/98]. Zur Normierung der Messwerte, deren Angabe in Licht-Einheiten erfolgt, kann durch die eingesetzte Proteinmenge in µg geteilt und das Ergebnis in Relativen-Licht-Einheiten (RLE) angegeben werden.

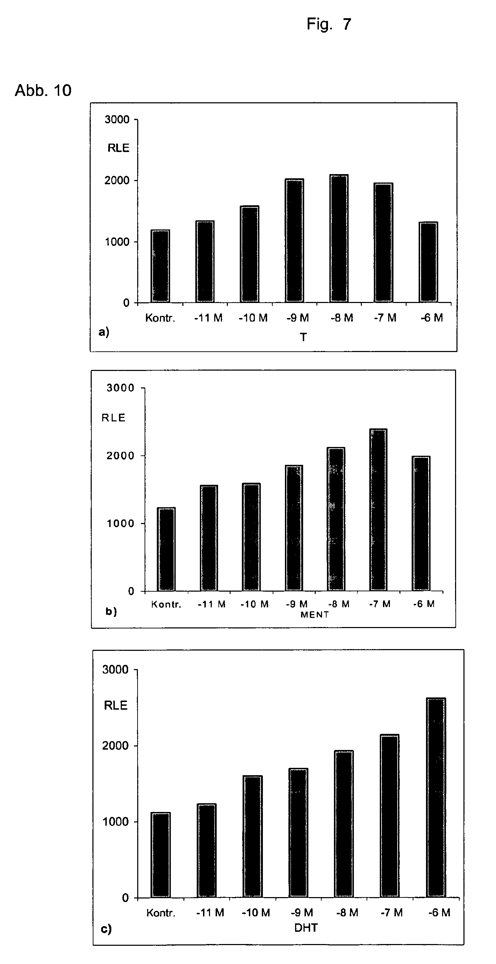

Das 1f-Reportergenprodukt zeigt in den durchgeführten Transfektion in der Prostata-Zell-Linie PC3 eine Induktion der Reportergenexpression mit DHT um den Faktor 2,6 (Abb. 7). Es kann hier die Regulation durch Androgene *in vitro* ermittelt werden.

### 3. Androgenabhängige Regulation des 1f-Aromatasepromotors

Zusätzlich zu den Prostata PC3-Zellen wird eine neuronale Zell-Linie verwendet zur Analyse von androgenregulierter Reportergenexpression am 1f-Aromatasepromotor.

Die humane Neuroblastoma Zell-Linie SH-SY5Y (von DSMZ GmbH, Braunschweig) wird in Dulbecco's MOD Eagle Medium mit Phenolrot (Bst.Nr.: 31885-023 von Gibco) + 15 % fötales Kälberserum (FKS) mit 5 mg/l Penicillin/Streptomycin passagiert und bei 37 ° C mit 5 % CO₂ und 100 % relativer Luftfeuchte in Brutschränken (Heraeus) kultiviert. Vor den Testungen werden die SH-SY5Y-Zellen zweimal mit Testmedium passagiert, dieses enthält kein Phenolrot (Bst.Nr.: 11880-028 von Gibco) und fötales Kälberserum, welches zuvor über Aktivkohle von Hormonresten gereinigt wurde (DCS).

Um den Einfluss von Hormonen auf den klonierten 1f-Aromatasepromotor in dem pGI3-Fusions-vektor 1f-pGI3 anhand der Luciferase-Aktivität zu bestimmen, werden in 6well Platten pro well 300000 Zellen mit 0,2 µg dieses Plasmides mit 0,5 µg des pSG5AR-Plasmids cotransfektiert. Der pSG5AR Vektor basiert auf den pSG5-Vektor, der über den SV40-early-gene-Promotor verfügt, der in humanen Zellen zur konstitutiven Expression der dahinter liegenden Gene führt (Breathnach, R. & Harris, B.A. 1983; "Plasmids for the cloning and expression of full-length double-stranded cDNAs under control of the SV40 early or late gene promotor", Nucleic Acids Res. 11:7119-36), in diesem Fall des Gens für den AR. Die transiente Transfektion mit dem Reportergenplasmid und dem pSG5AR-plasmid erfolgt mit Lipofectin nach den Angaben von GibcoBRL [Lipofectin-Reagent Packungsbeilage, Kat.Nr.: 18292-037]. 20 h nach der Transfektion wird das Androgen Dihydrotestosteron (DHT) zugegeben. Nach weiteren 24 Stunden Inkubation im Brutschrank werden die Zellen mit PBS gewaschen, lysiert und die Reportergenaktivität bestimmt. Die Bestimmung der Luciferaseaktivität erfolgt mit Luciferase-Assay-Substrat nach den Angaben von Promega [Luciferase-Assay-Substrat Packungsbeilage, Kat.Nr.: E151A aus: Luciferase Assay System with Reporter Lysis Buffer; Part TB161, USA 3/98]. Zur Normierung der Messwerte, deren Angabe in Licht-Einheiten erfolgte, kann durch die eingesetzte Proteinmenge in µg geteilt und das Ergebnis in Relativen-Licht-Einheiten (RLE) angegeben werden.

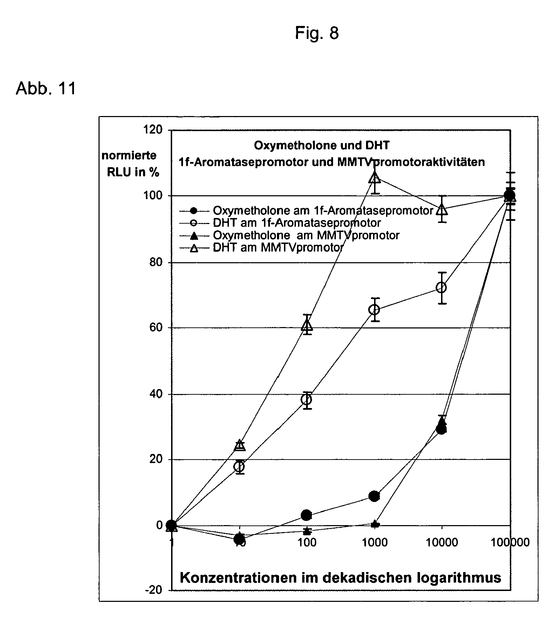

Das 1f-Reportergenprodukt zeigt in der durchgeführten Transfektion in der Neuroblastoma-Zell-Linie SH-SY5Y eine konzentrationsabhängige Induktion der Reportergenexpression mit DHT (Abb.8).
Da in beiden verwendeten Zell-Linien ähnliche Effekte gemessen werden, wird die Aktivität des 1f-Promotors in diesen Testsystemen unabhängig vom zellulären Kontext durch Androgene stimuliert.

### 4. Transfektionsexperimente zur Charakterisierung der hormonellen Regulation des 1f-Aromatasepromotors durch pharmakologisch aktive Substanzen

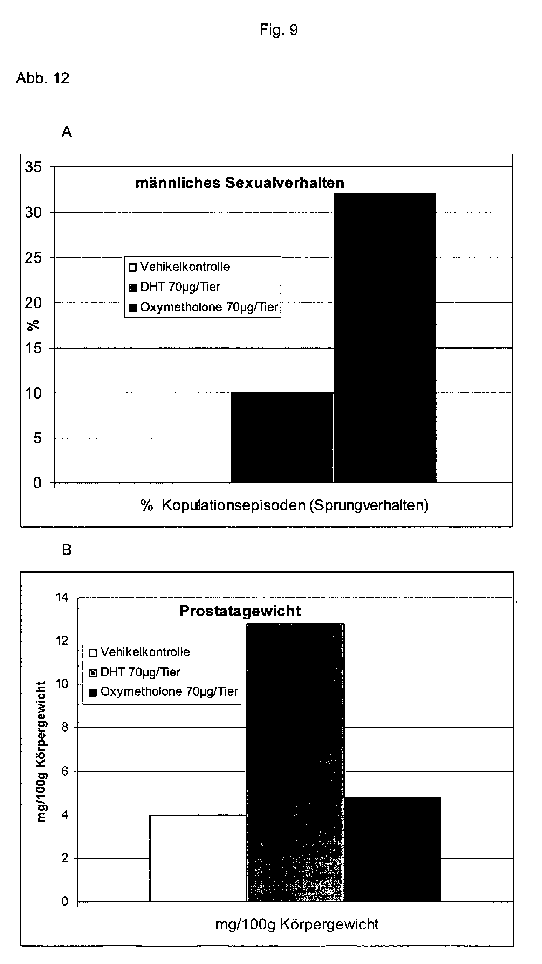

Neben DHT wird anhand des 1f-Luciferase-Reportergen-Konstruktes (1f-pGI3) als weitere pharmakologisch aktive Substanz der Einfluss von Estradiol auf die Expression der humanen Aromatase in neuronalen Geweben getestet (Abb. 9). PC3 (humane prostata carcinoma)-Zellen (von DSMZ GmbH, Braunschweig) wurden in RPMI 1640 Medium mit Phenolrot (Bst.Nr.: 31870-025 von Gibco) + 10 % fötales Kälberserum (FKS) mit 200 mM Glutamin und 5 mg/l Penicillin/Streptomycin passagiert und bei 37 ° C mit 5 % CO2 und 100 % relativer Luftfeuchte in Brutschränken (Heraeus) kultiviert. Vor den Testungen werden die PC3-Zellen zweimal mit Testmedium passagiert, dieses enthält kein Phenolrot (Bst.Nr.: 32404-014 von Gibco) und fötales Kälberserum, welches zuvor über Aktivkohle von Hormonresten gereinigt wurde (DCS).

Um den Einfluss von Estradiol (E2) auf den klonierten 1f-Aromatasepromotor in dem pGl3-Fusions-vektor 1f-pGl3 anhand der Luciferase-Aktivität zu bestimmen, werden in 6well Platten pro well 200000 Zellen mit 0,2 µg dieses Plasmides mit 0,5 µg des pSG5-ER-Plasmid cotransfektiert. Der pSG5-ER Vektor basiert auf dem pSG5-Vektor, der über den SV40-early-gene-Promotor verfügt, der in humanen Zellen zur konstitutiven Expression der dahinter liegenden Gene führt (Breathnach, R. & Harris, B.A. 1983; "Plasmids for the cloning and expression of full-length double-stranded cDNAs under control of the SV40 early or late gene promotor", Nucleic Acids Res. 11:7119-36), in diesem Fall des Gens für den humanen estrogen receptor alpha (ERα). Die transiente Transfektion mit dem Reportergenplasmid und dem pSG5-ER-plasmid erfolgt mit Lipofectin nach den Angaben von GibcoBRL [Lipofectin-Reagent Packungsbeilage, Kat.Nr.: 18292-037]. 20 h nach der Transfektion wird das Androgen Dihydrotestosteron (DHT) zugegeben. Nach weiteren 24 Stunden Inkubation im Brutschrank werden die Zellen mit PBS gewaschen, lysiert und die Reportergenaktivität bestimmt. Die Bestimmung der Luciferaseaktivität erfolgt mit Luciferase-Assay-Substrat nach den Angaben von Promega [Luciferase-Assay-Substrat Packungsbeilage, Kat.Nr.: E151A aus: Luciferase Assay System with Reporter Lysis Buffer; Part TB161, USA 3/98]. Zur Normierung der Messwerte, deren Angabe in Licht-Einheiten erfolgt, kann durch die eingesetzte Proteinmenge in µg geteilt und das Ergebnis in Relativen-Licht-Einheiten (RLE) angegeben werden.

Eine direkte Beeinflussung durch Estrogene auf die neuronale Aromataseexpression oder Aktivität ist in der Literatur nicht beschrieben. Auch in dem erfindungsgemäßen 1f-Reprotergenassay kann kein Effekt von Estradiol auf die 1f-Promotoraktivität gemessen werden (Abb. 9). Dieser Befund deckt sich auch mit einer Sequenzanalyse des 1f-Promotors, in der zumindest keine bekannten ERE-Sequenzen enthalten sind. Es wird in der Literatur zwar ein Einfluss der Estrogenkonzentration auf die Aromataseaktivität im Gehirn diskutiert (Rosselli, C. E. and Resko, J. A. (1993): Aromatase activity in the rat brain: hormonal regulation and sex differences; J. Steroid. Biochem., Vol. 44: 499-508), allerdings dahingehend, das dieser Einfluss über einen Feedback-Mechanismus vermittelt wird. Die von der Aromatase produzierten Estrogene senken die Expression der Androgenrezeptoren und damit indirekt auch die Aromataseexpression, da zur Wirkungsentfaltung der Androgenwirkung das Vorhandensein des Androgenrezeptors essentiell ist. Diese Feedback-Regulation ist bei dem erfindungsgemäßen *in vitro* Reportergenassay nicht gegeben, da die auf den pSG5-Vektor basierenden Rezeptorgen-Plasmide unabhängig von der Estrogenkonzentration zur Expression der Rezeptoren führen. Dieses Ausschalten der im komplexen *in vivo* System vorliegenden Feedback-Mechanismen erlaubt in den durchgeführten Transaktivierungsassays eine Untersuchung der direkten Regulation der Aromataseexpression auf Promotorebene. Zur weiteren genaueren Aufklärung der nötigen Stimuli für die direkte Regulation der neuronalen Aromataseexpression ist der erfindungsgemäße Reportergenassay daher bestens geeignet.

### 5. Transfektionsexperimente zur Suche und Charakterisierung von pharmakologisch aktiven Substanzen, welche die neuronale Aromataseexpression regulieren

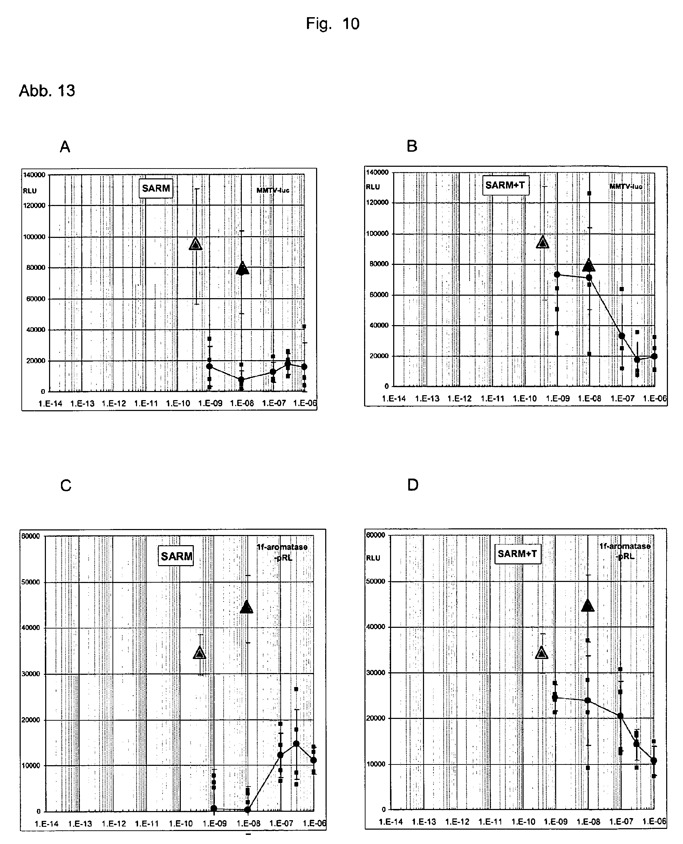

Neben DHT werden weitere pharmakologisch aktive Androgene mit dem 1f-Luciferase-Reportergen-Konstrukt getestet (Abb. 10).

Die humane Neuroblastoma Zell-Linie SH-SY5Y (von DSMZ GmbH, Braunschweig) wurde in Dulbecco's MOD Eagle Medium mit Phenolrot (Bst.Nr.: 31885-023 von Gibco) + 15 % fötales Kälberserum (FKS) mit 5 mg/l Penicillin/Streptomycin passagiert und bei 37 °C mit 5 % CO₂ und 100 % relativer Luftfeuchte in Brutschränken (Heraeus) kultiviert. Vor den Testungen werden die SH-SY5Y-Zellen zweimal mit Testmedium passagiert, dieses enthält kein Phenolrot (Bst.Nr.: 11880-028 von Gibco) und fötales Kälberserum, welches zuvor über Aktivkohle von Hormonresten gereinigt wurde (DCS).

Um den Einfluss von Hormonen auf den klonierten 1f-Aromatasepromotor in dem pGI3-Fusions-vektor 1f-pGI3 anhand der Luciferase-Aktivität zu bestimmen, werden in 6well Platten pro well 300000 Zellen mit 0,4 µg dieses Plasmides mit 0,5 µg des pSG5AR-Plasmids cotransfektiert. Der pSG5AR Vektor basiert auf dem pSG5-Vektor, der über den SV40-early-gene-Promotor verfügt, der in humanen Zellen zur konstitutiven Expression der dahinter liegenden Gene führt (Breathnach, R. & Harris, B.A. 1983; "Plasmids for the cloning and expression of full-length double-stranded cDNAs under control of the SV40 early or late gene promotor", Nucleic Acids Res. 11:7119-36), in diesem Fall das Gen für den AR. Die transiente Transfektion mit dem Reportergenplasmid und dem pSG5AR-Plasmid erfolgt mit Lipofectin nach den Angaben von GibcoBRL [Lipofectin-Reagent Packungsbeilage, Kat.Nr.: 18292-037]. 20 h nach der Transfektion werden die Testsubstanzen zugegeben. Nach weiteren 24 Stunden Inkubation im Brutschrank werden die Zellen mit PBS gewaschen, lysiert und die Reportergenaktivität bestimmt. Die Bestimmung der Luciferaseaktivität erfolgt mit Luciferase-Assay-Substrat nach den Angaben von Promega [Luciferase-Assay-Substrat Packungsbeilage, Kat.Nr.: E151A aus: Luciferase Assay System with Reporter Lysis Buffer; Part TB161, USA 3/98]. Zur Normierung der Messwerte, deren Angabe in Licht-Einheiten erfolgt, kann durch die eingesetzte Proteinmenge in µg geteilt und das Ergebnis in Relativen-Licht-Einheiten (RLE) angegeben werden.

Das 1f-Reportergenplasmid zeigt in der durchgeführten Transfektion eine konzentrationsabhängige Induktion der Reportergenexpression mit DHT. (Abb.8). Neben DHT werden auch andere Androgene mit dem 1f-Luciferase-Reportergen-Konstrukt getestet (Abb. 10). Alle Androgene stimulieren den 1f-Promotor, allerdings mit jeweils leicht unterschiedlicher Potenz. Eine Aktivierung kann auch mit MENT und Testosteronpropionat beobachtet werden. Die im relativen Vergleich sehr potente Stimulation durch Testosteronpropionat ist in Übereinstimmung mit den guten klinischen Effekten von Testosteronderivaten auf die männliche Libido (Gooren, 1987, Arch. Sex. Beh. 16:463-473; Bancroft 1988, in Handbook of Sexology 6, 297-315 Elsevier, Amsterdam, Buena et al., 1993, Fertil. Steril. 59: 1118-1123), welche im Primaten maßgeblich durch die Aktivierung der neuronalen Aromatasexpression gesteuert wird (Zumpe et al., 1993, Horm. Behav. 27:200-215).

### 6. Transfektionsexperimente zur Suche und Charakterisierung von gewebeselektiven Androgenen, die AR regulierte Gene im neuronalen Gewebe im Vergleich zu anderen Geweben unterschiedlich regulieren

Das erfindungsgemäße Testsystem mit dem neuronalen 1f-Aromatase-promotor wird kombiniert mit dem MMTV-Promotor, der eine allgemeine Androgenwirkung widerspiegelt (Parker, M.G. et al., J Cell Biochem. 1987, 35:285-92). Der hormonregulierte MMTV-promotor wird vor das Firefly-luciferase-Gen des pGL3control Vektors von Promega kloniert, um den Vektor MMTV-luc herzustellen.

Die humane Neuroblastoma Zell-Linie SH-SY5Y (von DSMZ GmbH, Braunschweig) wird in Dulbecco's MOD Eagle Medium mit Phenolrot (Bst.Nr.: 31885-023 von Gibco) + 15 % fötales Kälberserum (FKS) mit 5 mg/l Penicillin/Streptomycin passagiert und bei 37 ° C mit 5 % CO2 und 100 % relativer Luftfeuchte in Brutschränken (Heraeus) kultiviert. Vor den Testungen werden die SH-SY5Y-Zellen zweimal mit Testmedium passagiert, dieses enthält kein Phenolrot (Bst.Nr.: 11880-028 von Gibco) und fötales Kälberserum, welches zuvor über Aktivkohle von Hormonresten gereinigt wurde (DCS).

Um den Einfluss von Hormonen auf den klonierten 1f-Aromatasepromotor in dem pGl3-Fusions-vektor anhand der Luciferase-Aktivität im Vergleich zum MMTV-Promotor zu bestimmen, werden in parallelen Transfektionsexperimenten 1,5 µg des 1f-pGl3 Plasmides bzw. des MMTV-luc-Plasmides zusammen mit 0,75 µg des pSG5AR Plasmides in 300000 SH-SY5Y Zellen pro well einer 6well-Platte transfektiert. Die transienten Transfektion mit den Reportergenplasmiden erfolgt mit Lipofectin nach den Angaben von GibcoBRL [Lipofectin-Reagent Packungsbeilage, Kat.Nr.: 18292-037]. 20 h nach der Transfektion werden die Testsubstanzen zugegeben. Nach weiteren 24 Stunden Inkubation im Brutschrank werden die Zellen mit PBS gewaschen, lysiert und die Reportergenaktivität bestimmt. Die Bestimmung der Luciferaseaktivität erfolgt mit Luciferase-Assay-Substrat nach den Angaben von Promega [Luciferase-Assay-Substrat Packungsbeilage, Kat.Nr.: E151A aus: Luciferase Assay System with Reporter Lysis Buffer; Part TB161, USA 3/98]. Zur Normierung der Messwerte, deren Angabe in Licht-Einheiten erfolgte, wird durch die eingesetzte Proteinmenge in µg geteilt und die relativen Lichteinheiten pro µg eingesetztes Protein werden in % der maximalen Stimulation angegeben.

Das 1f-Reportergenplasmid zeigt in den durchgeführten Transfektionen in der Neuroblastoma-Zell-Linie SH-SY5Y eine Induktion der Reportergenexpression mit den getesteten Androgenen, in diesem Beispiel DHT und Oxymetholone. Auch das MMTV-Reportergenplasmid zeigt in beiden Fällen eine Induktion der Reportergenexpression (Abb. 12). Oxymetholone ist wie erwartet schwächer als DHT. Überraschenderweise und nicht vorhersehbar unterscheiden sich die Substanzen bezüglich der Induktionen an den beiden Promotorkonstrukten z.T. erheblich (Abb. 11). Während Oxymetholone an beiden getesteten Promotoren eine sehr ähnliche Aktivierung hervorruft, zeigt DHT deutliche Unterschiede zwischen den beiden Promotoren und ist am neuronalen 1f-Aromatasepromotor weniger potent im Vergleich zum MMTV Promotor. Diese Unterschiede weisen auf einen möglichen gewebeselektiven Unterschied *in vivo* und damit im Menschen hin. Aus Tierversuchen ist bekannt, dass DHT im Vergleich zu z.B. Testosteron wesentlich geringere Wirkungen auf das männliche Sexualverhalten hat, während es an der Prostata als sehr starkes Androgen wirkt (Arteaga-Silva, M. et al., 2005, Physiol Behav. 85: 571-80). Dieses Verhalten von DHT wurde nochmals im Vergleich zu Oxandrolon in einem aufwendigen *in vivo* Experiment, welches über 4 Monate lief, bestätigt (Abb. 12). Das erfindungsgemäße *in vitro* Testsystem erlaubt daher auf eine einfache und elegante Weise eine schnelle Suche und Charakterisierung von Androgenen mit einer möglichen gewebeselektiven Wirkung. Er stellt somit ein ideales Testsystem dar, mit dem Potential von Hochdurchsatztestungen.

### 7. Transfektionsexperiment zur simultanen Suche und Charakterisierung, auch bezüglich partialagonistischer und partialantagonistischer Wirkungen von gewebeselektiven Androgenen, die AR regulierte Gene gewebeselektiv differenziell regulieren

Das erfindungsgemäße Testsystem mit dem neuronalen 1f-Aromatase-promotor wird kombiniert mit dem MMTV-Promotor, der eine allgemeine Androgenwirkung widerspiegelt (Parker, M.G. et al., J Cell Biochem. 1987, 35:285-92). Beide androgenregulierten Promotoraktivitäten können simultan in einem Transfektionsansatz oder optional in einer stabil transfektierten Zell-Linie gemessen werden, bei der Verwendung von zwei unterschiedlichen Reportergenkonstrukten. In dem vorliegenden Beispiel wird der hormonregulierte MMTV-promotor vor das Firefly-luciferase-Gen des pGL3control Vektors von Promega kloniert, um den Vektor MMTV-luc herzustellen, während der 1 f-Aromatasepromotor vor das Renilla-luciferase-Gen des pRL-vektors von Promega kloniert wird, um den Vektor 1f-Aromatase-pRL herzustellen.

Zur optionalen Vereinfachung des experimentellen Ansatzes wird der AR zunächst stabil in einer Zell-Linie transfektiert. Die cDNA des AR wird in das Expressionsplasmid pSG5 kloniert, um das Plasmid pSG5AR zu generieren. Dieser Vektor verfügt über den SV40-early-gene-Promotor, der in humanen Zellen zur konstitutiven Expression der dahinter liegenden Gene führt (Breathnach, R. und Harris, B.A. 1983, Nucleic Acids Res. 11:7119-36), in diesem Fall das Gen des AR. PC3 (humane prostata carcinoma)-Zellen (von DSMZ GmbH, Braunschweig) werden stabil mit dem pSG5AR Vektor transfektiert, unter zusätzlicher Verwendung eines Plasmids, welches ein Resistenzgen für das Antibiotikum G418 kodiert. Die resultierenden PC3-AR+ Zellen exprimieren stabil den humanen AR. PC3AR+ Zellen werden in RPMI 1640 Medium mit Phenolrot (Bst.Nr.: 31870-025 von Gibco) + 10 % fötales Kälberserum (FKS) mit 200 mM Glutamin, 5 mg/l Penicillin/Streptomycin und 200µg/ml Geniticin passagiert und bei 37 ° C mit 5 % CO₂ und 100 % relativer Luftfeuchte in Brutschränken (Heraeus) kultiviert. Vor den Testungen wurden die PC3-AR+ zweimal mit Testmedium passagiert, dieses enthält kein Geniticin, kein Phenolrot (Bst.Nr.: 32404-014 von Gibco) und fötales Kälberserum, welches über Aktivkohle von Hormonresten gereinigt wird (DCS).

Um den Einfluss von Hormonen auf den klonierten 1f-Aromatasepromotor in dem pRL-Fusions-vektor anhand der Renilla-Aktivität im Vergleich zur Firefly-Luciferaseaktivität des MMTV-Promotors zu bestimmen, werden in Transfektionsexperimenten 8 µg des 1f-aromatase-pRL Plasmides und 20 µg des MMTV-luc-Plasmides in 300000 PC3-AR+ Zellen pro 175ml Flasche transfektiert. Die transienten Transfektion mit den Reportergenplasmiden erfolgt mit Lipofectin nach den Angaben von GibcoBRL [Lipofectin-Reagent Packungsbeilage, Kat.Nr.: 18292-037]. 6h nach Transfektion werden die Zellen gewaschen und auf 96well Mikrotiterplatten verteilt, mit 10000 Zellen pro well. 16 h nach der Umsetzung auf 96well Platten wird ein selektiver Androgenrezeptor Modulator (SARM) zugegeben, sowohl alleine zur Messung der agonistischen Aktivitäten als auch in Anwesenheit von 10 x e⁻¹⁰ M Testosteron zur Messung der antagonistischen Aktivitäten. Nach weiteren 20 Stunden Inkubation im Brutschrank werden die Zellen mit PBS gewaschen, lysiert und die Reportergenaktivitäten bestimmt. Die Bestimmung der Firefly-und Renilla-Luciferaseaktivitäten in relativen Lichteinheiten erfolgt mit einem dualen Luciferase-Assay-Kit (Best.Nr.: E 1960 von Promega) nach den Angaben des Herstellers [Promega; Luciferase-Assay-Substrat Packungsbeilage, Kat.Nr.: E151A aus: Luciferase Assay System with Reporter Lysis Buffer; Part TB161, USA 3/98]. Zur optionalen Normierung der Messwerte, deren Angabe in Licht-Einheiten erfolgte, kann durch die eingesetzte Proteinmenge in µg geteilt und das Ergebnis in Relativen-Licht-Einheiten (RLE) angegeben werden.

Der hier getestete SARM wirkt im Test auf Agonismus, wie schon das im vorigen Beispiel aufgeführte DHT, deutlich unterschiedlich auf die beiden ARabhängigen Promotoren (Abb. 13). Im Unterschied zu DHT erfolgt hier aber keinerlei Aktivierung des allgemeinen MMTV Promotors, während der neuronale 1f-Aromatasepromotor, der als Beispielpromotor eines AR regulierten Promotors außerhalb der Gonaden steht, aktiviert wird. Dies deutet, wie das vorherige Beispiele mit DHT, auf eine mögliche Gewebeselektivität auch *in vivo* und im Menschen hin. Darüber hinaus können durch die parallelen Messungen des Antagonismus Aussagen über die mögliche selektive Modulation der AR-Aktivität gewonnen werden. D.h. über den Stand der Technik hinaus können Aussagen zur SARM-Aktivität bezüglich einer möglichen gewebeselektiven Wirkung gewonnen werden.

Wie in diesem Beispiel auch deutlich gemacht, können die Messungen im Mikrotiterformat erfolgen, was die prinzipielle Hochdurchsatzfähigkeit des erfindungsgemäßen Testsystems unterstreicht. SARMs, welche wie in diesem Beispiel im Allgemeinen klar antagonistisch wirken, jedoch in spezifischen Geweben auch eine gewisse agonistische Wirkung zeigen, sind von besonderem Interesse für verschiedenste Indikationen (S.S.Wolf und M.Obendorf, 2004, in E. Nieschlag & H. M. Behre: 'Testosterone' 3rd. Edition; Cambridge University press, ISBN 0521833809; 623-640).

### Abbildungen

### Abbildung 1:

Genomischer Promotorbereich des humanen Aromatasegens mit den bekannten Splicing-Varianten der alternativen Exon-I-Varianten (vereinfachte Darstellung). Zur besseren Übersicht sind Teile des 5'-Bereiches ausgeblendet und durch Auslassungen angegeben (modifiziert nach Harada, N. et al., Proc. Natl. Acad. Sci. USA, Vol. 90: 11312-11316, 1993 und Simpson, E. R.; Endocrine Reviews, Vol. 15: 341-355, 1994).

### Abbildung 2:

PCR-Produkt mit dem 1f-aro-promotor, amplifiziert aus genomischer DNA von SH-SY5Y-Zellen. a: Längenstandard b: PCR Amplifikat von 1f-Aromatase promotor.

### Abbildung 3:

Karte des pTAdv-Vektors. Dieser linearisierte und mit einem 3'-T-Überhang an beiden Enden versehene Vektor wurde für die Ligationen mit PCR-Fragmenten eingesetzt.

### Abbildung 4:

Vektorkarte des 1f-pTadv" vektors mit der amplifizierten 1f-aromatasepromotor-DNA im pTAdv-Vektor. HindIII und Xhol markieren Schnittstellen, die zur weiteren Klonierung verwendet wurden.

### Abbildung 5:

Vektorkarte des pGl3-control-Vektors. *Hind*III und Xhol markieren Schnittstellen, die zur weiteren Klonierung verwendet wurden.

### Abbildung 6:

Vektorkarte des 1f-pGl3-Vektors. HindIII und Xhol markieren Schnittstellen, die zur Herstellung des Vektors verwendet wurden.

### Abbildung 7:

Abhängigkeit der Aktivierung des 1f-Promotors von der Konzentration an Dihydrotestosteron (DHT) in PC3-Zellen Es werden 0,2µg des 1f-pGI3-Plasmides und 0,5µg des pSG5-AR-Plasmides transfektiert. Die Inkubation mit DHT erfolgt für 20h. Dargestellt sind die Mittelwerte der Doppelbestimmungen in Relativen-Licht-Einheiten (RLE) pro µg Gesamtprotein.

### Abbildung 8:

Abhängigkeit der Aktivierung des 1f-Promotors von der Konzentration an Dihydrotestosteron (DHT) in SH-SY5Y-Zellen. Es werden 0,2µg des 1f-pGI3-Plasmides und 0,5µg des pSG5-AR-Plasmides transfektiert. Die Inkubation mit DHT erfolgt für 20h. Dargestellt sind die Mittelwerte der Doppelbestimmungen in Relativen-Licht-Einheiten (RLE) pro µg Gesamtprotein.

### Abbildung 9:

Unabhängigkeit der Aktivierung des 1f-Promotors von der Konzentration an Estradiol (E2) in PC3-Zellen. Es werden 0,2µg des 1f-pGl3-Plasmides und 0,5µg des pSG5-ER-Plasmides transfektiert. Die Inkubation mit E2 erfolgt für 20h. Dargestellt sind die Mittelwerte der Doppelbestimmungen in Relativen-Licht-Einheiten (RLE) pro µg Gesamtprotein.

### Abbildung 10:

Einfluss verschiedener Androgene auf den 1f-Promotor in neuronalen SH-SY5Y-Zellen. Es werden 0,4 µg des 1f-pGl3 und 0,5 µg des pSG5-AR-Plasmides transfektiert.
Die Inkubation mit a) T (Testosteronpropionat), b) MENT (7α-Methyl-19-nortestosteron) und c) DHT (Dihydrotestosteron) erfolgt für 20h. Es werden die Mittelwerte aus den Doppelbestimmungen in Relativen-Licht-Einheiten (RLE) pro µg Gesamtprotein dargestellt.

### Abbildung 11:

Einfluss verschiedener Androgene auf den 1f-Promotor und den MMTV-Promotor in neuronalen SH-SY5Y-Zellen. Es werden 1,5 µg des 1f-pGI3 und des MMTV-luc promotors sowie 0,75µg des pSG5-AR-Plasmides transfektiert. Die Inkubation mit Oxymetholone oder DHT (Dihydrotestosteron) erfolgt für 20h. Es werden die normierten Mittelwerte der Relativen-Licht-Einheiten (RLU) in % aus den Vierfachbestimmungen mit Standardabweichungen dargestellt.

### Abbildung 12:

*In vivo* Einfluss verschiedener Androgene auf das männliche Sexualverhalten (A), welches einen neuronalen Endpunkt der Androgenwirkung darstellt und auf das Prostatagewicht (B), welches einen gonadalen Endpunkt der Androgenwirkung darstellt. Intakte männliche Ratten mit sexueller Erfahrung werden ausgewählt und kastriert. Nach 7 Wochen und Verlust der sexuellen Aktivität werden die Tiere in drei Gruppen eingeteilt mit je 9 - 10 Tieren pro Gruppe und eine Woche mit 1050 µg Oxymetholone, DHT oder Vehikel behandelt. Von Tag 8 bis Tag 120 erhalten die Tiere täglich 70 µg Oxymetholone (schwarz), DHT (dunkelgrau) oder Vehikel (hellgrau). Jedes Tier wurde 7 mal auf Sexualverhalten (Sprungverhalten) getestet. 4 Monate nach Beginn der Studie wurden die Organe entnommen und Prostata und Körpergewichte bestimmt.

### Abbildung 13:

Testung auf Agonismus (A+C) und Antagonismus (B+D) eines selektiven AR Modulators (SARM) simultan am unspezifischen MMTV-promotor (A+B) und am neuronalen 1f-Aromatasepromotor (C+D) in PC3AR+-Zellen. Es werden 1,5 µg des 1f-pGl3 und des MMTV-luc promotors sowie 0,75 µg des pSG5-AR-Plasmides transfektiert.
Die Inkubation mit dem SARM erfolgt für 20 h. Bei der Testung auf Antagonismus (B+D) wird der SARM in den angegebenen Konzentrationen in der Anwesenheit von 4x10 e⁻¹⁰ M Testosteron inkubiert. Es werden die Einzelwerte quadratisch und die Mittelwerte der relativen Lichteinheiten aus Vierfachbestimmungen kreisförmig mit Standardabweichungen dargestellt. Als Dreiecke dargestellt sind die Werte für Testosteron ohne Zugabe von dem SARM. Am unspezifischen MMTV-promotor (A+B) verhält sich der hier getestete SARM als reiner Antagonist der AR vermittelten Transaktivierung, während am neuronalen 1f-Aromatasepromotor (C+D) die partial agonistische/partial antagonistische Wirkung hervortritt.

## Patentansprüche

1. Promotor-Reportergenfusion bestehend aus 1f-Aromatase-Promotor gemäß Seq ID #1 und Reportergen.

2. Promotor-Reportergenfusion gemäß Anspruch 1, wobei das Reportergen ausgewählt sein kann aus der Gruppe umfassend ein Firefly- oder Renilla-Luziferasegen und wobei das Fusionsprodukt stabil oder transient exprimiert werden kann in eine Zell-Linie.

3. Verwendung der Promotor-Reportergenfusion gemäß Anspruch 1-2, **dadurch gekennzeichnet, dass** in einem Zellkultursystem Substanzen identifiziert werden können, die über Bindung an den Androgenrezeptor die Aromataseregulation in neuronalen Geweben beeinflussen.

4. Verwendung gemäß Anspruch 3, wobei Sequenzen, die > 90 %, vorzugsweise > 95 % Homologie aufweisen, zu der unter Seq ID # 1 angegebenen Sequenz in einem Zellkultursystem zur Identifikation von Substanzen eingesetzt werden, die über Bindung an den Androgenrezeptor die Aromataseregulation im Gehirn beeinflussen.

5. Bestimmung der gewebsselektiven Wirkung von Substanzen, die an den Androgenrezeptor binden, umfassend folgende Schritte:
a. Bestimmung der Modulation der Aromataseregulation unter Verwendung der Promotor-Reportergenfusion in einem Reportergenassay in Zellkultursystemen zur Identifikation von agonistischen wie auch antagonistischen Aktivitäten,
b. Bestimmung der Modulation eines anderen Androgen-regulierten Promotors und
c. Vergleich von Schritt a und b, um eine vollständige *in vitro* Charakterisierung von Substanzen bezüglich selektiver AR Modulation am 1f-Aromatasepromotor oder im direkten Vergleich mit anderen AR regulierten Promotoren zu erzielen.

6. Bestimmung der gewebsselektiven Wirkung von Substanzen gemäß Anspruch 5, wobei im Schritt a) Sequenzen, die >90%, vorzugsweise >95 % Homologie aufweisen zu der unter Seq ID # 1 angegebenen Sequenz verwendet werden.

7. *In vitro* Testsystem zur Identifizierung und Charakterisierung von Substanzen, die die Erhöhung der Aromataseexpression selektiv im zentralen Nervensystem bewirken und/oder geeignet sind, die Aktivität des AR gewebeselektiv zu beeinflussen, wobei das Testsystem eine Fusion aus Reportergen und Aromatasepromotor gemäß Anspruch 1 oder 2 enthält.
